# EUROPÄISCHE PATENTANMELDUNG

(11) **EP 3 476 757 A1**
(43) Veröffentlichungstag der Anmeldung: **01.05.2019**
(21) Anmeldenummer: 17198826.4
(22) Anmeldetag: 27.10.2017
(51) Int. Cl.: B65D 23/12, A23L 33/105, A23L 33/15, A23L 33/16

(54) **KOMBINATIONSBEHÄLTNIS ZUR VERPACKUNG EINER FLÜSSIGKEIT UND EINER FESTEN SUBSTANZ**

(71) Anmelder: Beneganic GmbH, 8045 Zürich (CH)
(72) Erfinder: HARMS-GÄNSHIRT, Dorothee, 8842 ALTENDORF (CH); HARMS, Fred, 8852 ALTENDORF (CH)
(74) Vertreter: Dantz, Dirk

(57) **Zusammenfassung**

Die vorliegende Erfindung betrifft eine Darreichungsform eines festen und flüssigen Nahrungsergänzungsmittels, wobei das Nahrungsergänzungsmittel einen Multivitaminkomplex, sekundäre Pflanzenstoffe und produktspezifische Wirkstoffe aufweist und eine flüssige Komponente, eine erste feste Komponente und eine zweite feste Komponente umfasst.

## Beschreibung

Die Erfindung betrifft eine Darreichungsform für ein festes und flüssiges Nahrungsergänzungsmittel, das einen Multivitaminkomplex, sekundäre Pflanzenstoffe und produktspezifische Wirkstoffe aufweist.

Nahrungsergänzungsmittel sind Produkte zur ausreichenden Versorgung des menschlichen Stoffwechsels mit bestimmten z.T. essentiellen Nähr- oder Wirkstoffen im Grenzbereich zwischen Arzneimitteln und Lebensmitteln.

Eine andere Gruppe bilden die sekundären Pflanzenstoffe wie Amygdalin (Lätril) und Chlorophyll. Diese von Pflanzen produzierten Verbindungen spielen im menschlichen Organismus zwar keine überlebenswichtige Rolle, mehrere Studien, eine zunehmende Anzahl wissenschaftlicher Studien weist jedoch sekundären Pflanzenstoffen multiple gesundheitsfördernde Eigenschaften nach.

Konventionelle Vitaminpräparate und Nahrungsergänzungsmittel werden synthetisch hergestellt und diese werden in letzter Zeitzunehmend kritisch betrachtet. Große randomisierte Placebo-kontrollierte Studien und Metaanalysen belegen, dass synthetische Vitamine zum einen unvollständig sind, da sie nur einen kleinen Teil des organisch wirksamen Vitaminkomplexes kopieren, sondern dass sie sogar gesundheitsgefährdend sind. Es ist inzwischen wissenschaftlich belegt, dass synthetische Vitamine das Risiko für Herzinfarkt, Schlaganfälle und Krebs erhöhen können. Die herkömmlichen Nahrungsergänzungen bestehen überwiegend aus synthetisch hergestellten Vitaminen und nicht-organischen Mineralstoffen und Spurenelementen. Der Mensch ist jedoch über Millionen von Jahren an die Verarbeitung organischer Nahrung angepasst und sein Stoffwechsel ist dementsprechend auf organische Vitamine, Mineralstoffe und Spurenelemente optimiert. Unser Körper kann daher zwischen natürlichen und künstlichen Stoffen unterscheiden, was für manche Stoffe sogar wissenschaftlich belegt ist (Beispiel Vitamin E). Darum haben organische Vitamine eine optimale Wirkung und so gut wie keine Nebenwirkungen, wohingegen künstliche Vitaminpräparate nicht nur wegen ihrer Unvollständigkeit weniger wirkungsvoll sondern auch gesundheitsschädlich sein können. In synthetischen Vitaminpräparaten sind zudem oftmals Chemikalien, Farbstoffe, künstliche Süßstoffe, Aluminium, Verdickungsmittel, synthetische Antioxidantien, Aromen oder auch Parabene enthalten.

Es gibt zwar eine Vielzahl von Nahrungsergänzungsmitteln, die unterschiedliche Wirkungen zum Ziel haben. Die meisten sind jedoch synthetisch. Die wenigen 100% organischen Präparate beschränken sich entweder auch eines oder sehr wenige essentielle Vitamine. Auch beinhalten sie keine spezifischen Wirkstoffe, die eine über die Deckung des Grundbedarfs an Vitaminen und Mineralstoffen hinausgehendes Ziel haben. Eine weitere Gruppe organischer Präparate stellt jeweils nur einen oder einige sekundäre Pflanzenstoffe zur Verfügung ohne einen umfassenden Anteil an organischen Vitaminen. Eine fritte Gruppe organischer Präparate bietet lediglich Pflanzenextrakte, deren Wirkstoffkonzentration entweder nicht benannt wird, oder unterhalb der wirksamen Dosierung liegt. Zusätzlich gibt es Produkte pflanzlicher Herkunft, denen während des Herstellungsprozesses synthetische Vitamine und anorganische Mineralien zugesetzt werden, da der Anteil der organischen Vitamine zu gering ist. Organische Vitamine und Mineralien können nur in aufwändigen Verfahren extrahiert werden und besonders eine Anreicherung der essentiellen Vitamine und Mineralien auf eine wirkungsvolle Konzentration stellt eine Herausforderung dar. Ein

Es ist daher Aufgabe der vorliegenden Erfindung, ein Nahrungsergänzungsmittel bereitzustellen, dass sicherstellt, dass der Grundbedarf durch natürliche Vitamine, Mineralstoffe und Antioxidantien gedeckt ist, eine spezifische Wirkungskomponente aufweist und gleichzeitig in einer einzigen Dosierung angeboten werden kann.

Die erfindungsgemäße Aufgabe wird durch eine Vorrichtung gemäß Anspruch 1 gelöst.

Die erfindungsgemäße Darreichungsform umfasst eine flüssige Komponente, eine erste feste Komponente sowie eine zweite feste Komponente eines Nahrungsergänzungsmittels für den menschlichen Verzehr. Das Nahrungsergänzungsmittel selbst weist einen Multivitaminkomplex, sekundäre Pflanzenstoffe und produktspezifische Wirkstoffe auf. Der Multivitaminkomplex enthält alle essentiellen Vitamine, die der menschliche Körper benötigt und im Wesentlichen nicht selbst herstellen kann. Diese essentiellen Vitamine müssen daher von außen durch z.B. die Nahrung zugeführt werden. Häufig fehlt jedoch die Zeit für eine ausgewogene Ernährung bzw. das Wissen über eine solche. Nahrungsergänzungsmittel können diese Defizite ausgleichen und dem menschlichen Körper alle wichtigen Stoffe zuführen, die er zur Gesunderhaltung bzw. im Falle von chronischen Krankheiten benötigt. Sekundäre Pflanzenstoffe wie Polyphenole haben multiple positive Gesundheitseffekte; sie können den Blutzucker- und Cholesterin oder Blutzuckergehalt senken, sie wirken entzündungshemmend, können die Arterien vor Verkalkung schützen, wirken zum Teil stark antioxidativ und einige können das Krebsrisiko verringern. Produktspezifische Wirkstoffe werden der erfindungsgemäßen Darreichungsform hinzugesetzt, um je nach Wunsch des Anwenders eine spezifische Wirkung zu erzielen. Die produktspezifischen Wirkstoffe sind im Wesentlichen sekundäre Pflanzenstoffe. Um Alterserscheinungen des menschlichen Körpers entgegenzuwirken, können z.B. Q10 und Omega-3-Fettsäuren enthalten sein, die Haut und Blutgefäße des menschlichen Körpers schützen. Zur Stärkung des Immunsystems kann der Darreichungsform z.B. Grünteeextrakt als Antioxidans zugesetzt sein.

Weiterführende Ausbildungen der Erfindung zur Sensorvorrichtung sind in den Unteransprüchen 2 bis 16 dargelegt.

In einer vorteilhaften Gestaltung der Erfindung ist die erste feste Komponente in einer ersten Kapsel oder Tablette zusammengefasst, die zweite feste Komponente ist in einer zweiten Kapsel oder Tablette. Auf diese Weise ist es möglich, den festen Komponenten unterschiedliche Wirkstoffe hinzuzusetzen und unterschiedliche Wirkungen zu erzielen. So kann z.B. die erste Kapsel oder Tablette einen Multivitaminkomplex enthalten, der der menschliche Körper zur Gesunderhaltung benötigt. In der zweiten Kapsel oder Tablette können produktspezifische Wirkstoffe enthalten sein, die eine spezifische Wirkung je nach Wunsch des Anwenders erzielen.

In einer weiteren Ausbildung der Erfindung weist die flüssige Komponente sekundäre Pflanzenstoffe auf. Die sekundären Pflanzenstoffe wie Polyphenole in der Flüssigkeit sollen einen präventiven gesundheitlichen Effekt haben.

In einer weiteren Ausführung der Erfindung weisen die sekundären Pflanzenstoffe Extrakte von Acerola und/oder Granatapfel auf. In hoch dosierter Form erzielen die Polyphenole gerade dieser beiden Früchte eine positive Wirkung auf Blutzucker- und Cholesteringehalt sowie auf Entzündungen und können das Krebsrisiko mindern.

In einem weiteren Aspekt der Erfindung weist die flüssige Komponente mindestens 100 mg, bevorzugt 500 mg und besonders bevorzugt 750 mg sekundäre Pflanzenstoffe auf. Die besonders bevorzugte Menge von 750 mg sekundäre Pflanzenstoffe entspricht der aus ca. 1 kg Granatäpfeln extrahierbaren Menge von Polyphenolen. Diese hohe Konzentration in der flüssigen Komponente gewährleistet einen positiven Effekt auf Blutzucker- und Cholesteringehalt sowie auf Entzündungen und kann das Krebsrisiko mindern.

In einer weiteren Gestaltung der Erfindung weist die flüssige Komponente einen Stabilisator auf. Der Stabilisator stabilisiert die in der flüssigen Komponente enthaltenen Wirkstoffe auch über einen langen Zeitraum und gewährleistet damit die Wirksamkeit der flüssigen Komponente auch bei Anwendung nach längerer Lagerung.

In einer weiteren Ausführung der Erfindung ist der Stabilisator der flüssigen Komponente Vitamin C. Vitamin C stabilisiert nicht nur die Wirkstoffe in der flüssigen Komponente, sondern besitzt auch einen positiven Effekt auf das Immunsystem des menschlichen Körpers. Der Stabilisator ist also selbst auch ein Wirkstoff, zusätzliche Stabilisatoren wie z.B. synthetisch erzeugte Stabilisatoren sind durch diese vorteilhafte Gestaltung nicht nötig.

In einer weiteren Ausbildung der Erfindung weist die flüssige Komponente eine Konzentration von Vitamin C in Höhe von 1 g/l bis 15 g/l auf. Vitamin C besitzt einen starken Eigengeschmack, der in zu hoher Konzentration als unangenehm empfunden werden kann. Eine zu hohe Konzentration von Vitamin C kann bei Anwendung der flüssigen Komponente zu einem unangenehmen Geschmackserlebnis beim Anwender führen und ihn dazu verleiten, die flüssige Komponente nicht mehr anzuwenden. Die korrekte Wahl der Vitamin-C-Konzentration schließt dies weitgehend aus.

In einer weiteren Gestaltung der Erfindung weist die erste feste Komponente einen Multivitaminkomplex auf. Der Multivitaminkomplex enthält alle essentiellen Vitamine, die der menschliche Körper benötigt und im Wesentlichen nicht selbst herstellen kann. Diese essentiellen Vitamine müssen daher von außen durch z.B. die Nahrung zugeführt werden.

In einer besonders vorteilhaften Ausgestaltung der Erfindung ist der Multivitaminkomplex zu 100% organischer Herkunft. Es hat sich herausgestellt, dass synthetisch hergestellte Vitamine keine identischen Kopien der in der Natur vorkommenden Vitamine sind. Synthetisch hergestellte Vitamine unterscheiden sich in Struktur und Zusammensetzung von den organischen natürlich vorkommenden Originalen. Ein natürlich vorkommendes Vitamin besteht in der Regel aus mehreren Komponenten wie zusätzlichen Koenzymen und anderen Kofaktoren, die erst im Zusammenspiel ihre positive Wirkung im menschlichen Körper entfalten. Ein synthetisch hergestelltes Vitamin ist aber in der Regel ein einzelnes Molekül und enthält diese Zusatzstoffe nicht. Seine positive Wirkung auf den menschlichen Körper ist deshalb begrenzt. Der in der Erfindung verwendete Multivitaminkomplex aus organischer Herkunft, d.h. extrahiert aus natürlich vorkommenden Früchten, Pflanzen und Heilkräutern gewährleistet durch das Zusammenspiel von Vitaminen und deren Komponenten eine positive Wirkung auf den menschlichen Körper. Es hat sich außerdem gezeigt, dass synthetisch hergestellte Vitamine einen negativen Effekt auf den menschlichen Körper haben können. Studien belegen ein z.B. erhöhtes Krebsrisiko durch den Konsum von synthetisch herausgestellten Vitaminen. Der in der Erfindung verwendete Multivitaminkomplex aus organischer Herkunft hat eine ausschließlich positive Wirkung auf den menschlichen Körper.

In einer vorteilhaften Weiterbildung der Erfindung sind die sekundären Pflanzenstoffe, der Multivitaminkomplex, die Mineralstoffe und/oder die produktspezifischen Wirkstoffe rein organischer Herkunft.

In einer weiteren Ausbildung der Erfindung weist der Multivitaminkomplex die Vitamine B5 und D auf. In weiteren optionalen Ausgestaltung der Erfindung weist der Multivitaminkomplex auch die Vitamine B1, B2, B3, B6, B7, B9, C, E und Beta Carotin auf.

In einer weiteren Ausführung der Erfindung enthält der Multivitaminkomplex Mineralstoffe. Essentielle Mineralstoffe wie z.B. Eisen, Zink und Selen unterstützen Blutbildung, Stoffwechsel und Knochen- bzw. Knorpelbildung.

In einer weiteren Gestaltung der Erfindung umfassen die Mineralstoffe Calcium, Phosphor und/oder Kalium. Diese Mineralstoffe sind essentiell für Knochenbau bzw. den Stoffwechsel und können dem menschlichen Körper nur von außen zugeführt werden.

In einer weiteren vorteilhaften Ausführungsform der Erfindung weist die erste feste Komponente mindestens 100 mg, bevorzugt 200 mg und besonders bevorzugt 300 mg Mineralstoffe und/oder Multivitaminkomplex auf.

In einer vorteilhaften Ausgestaltung der Erfindung weist die zweite feste Komponente produktspezifische Wirkstoffe auf. Diese Ausgestaltung vereinfacht den Herstellungsprozess. Die flüssige und die erste feste Komponente besitzen in jeder Dosis jeweils die gleiche Zusammensetzung. Beide Komponenten können daher in einem standardisierten Verfahren kostengünstig hergestellt und in einem Behältnis verpackt werden. Um spezifische Wirkungen zu erzielen, wird die zweite feste Komponente nach ihrer Herstellung der Verpackung zugeführt. Auf diese Weise können Nahrungsergänzungsmittel mit z.B. Antioxidans-Wirkung oder zur Reduzierung von Alterserscheinungen zusammengestellt und entsprechend gekennzeichnet werden.

In einer weiteren Ausbildung der Erfindung sind die produktspezifischen Wirkstoffe wirksam zur Entgiftung, zum Schutz des Immunsystems, zum Schutz der Haut und/oder Gefäßgesundheit. Die in der zweiten festen Komponente enthaltenen spezifischen Wirkstoffe können also je nach der vom Anwender gewünschten Wirkung kombiniert und dosiert werden.

In einer weiteren Ausführung der Erfindung enthalten die produktspezifischen Wirkstoffe einen oder mehrere Wirkstoffe aus der Gruppe der Bestandteile Grünteeextrakt, Lycopin, Omega-3-Fettsäuren und anderer Antioxidantien. Diese spezifischen Wirkstoffe unterstützen das Immunsystem und schützen den menschlichen Körper durch ihre antioxidative Wirkung vor Umweltschadstoffen.

In einer weiteren vorteilhaften Ausführungsform der Erfindung weist die zweite feste Komponente mindestens 50 mg, bevorzugt 150 mg und besonders bevorzugt 200 mg produktspezifische Wirkstoffe auf.

In einer weiteren Gestaltung der Erfindung sind die erste feste Komponente und die zweite feste Komponente optisch unterscheidbar. Beide feste Komponenten können sich z.B. durch ihre Formgebung bzw. Farbe unterscheiden. So kann der Anwender zuverlässig erkennen, welche Komponente mit welchen Wirkstoffen er zu sich nimmt. Auch im Herstellungsprozess kann in einfacher Weise überwacht werde, dass die Bestückung der Nahrungsergänzungsmittel in der vorgesehenen Form erfolgt ist.

In einer vorteilhaften Ausgestaltung der Erfindung ist die erfindungsgemäße Darreichungsform mit einer flüssigen, einer ersten festen und einer zweiten festen Komponente in einem Kombinationsbehältnis verpackt. Das Kombinationsbehältnis umfasst ein erstes Behältnis, das dazu bestimmt ist, die flüssige Komponente aufzunehmen. Dieses erste Behältnis ist mittels eines Schraubverschlusses verschließbar. Der Schraubverschluss ist so ausgeführt, dass er ein zweites Behältnis für die beiden festen Substanzen bildet, das durch einen Deckel verschließbar ist. Diese Ausführung des Kombinationsbehältnisses ermöglicht eine getrennte Aufbewahrung der in den Behältnissen befindlichen, aber in der Anwendung zusammengehörenden Substanzen. Üblicherweise ist in dem Kombinationsbehältnis eine Tagesdosierung verpackt. Die bestimmungsgemäße gemeinsame Anwendung der zusammengehörigen Substanzen ist mit der zusammengefassten Aufbewahrung im Kombinationsbehältnis gewährleistet.

In einer weiteren vorteilhaften Ausgestaltung der Erfindung sind die Mineralstoffe, die sekundären Pflanzenstoffe und/oder die produktspezifischen Wirkstoffe zu 100% organischer Herkunft.

Ausführungsbeispiele der erfindungsgemäßen Sensorvorrichtung und des erfindungsgemäßen Verfahrens sind in den Zeichnungen schematisch vereinfacht dargestellt und werden in der nachfolgenden Beschreibung näher erläutert.

Es zeigen:
- Fig. 1: Seitenansicht der erfindungsgemäßen Darreichungsform im Kombinationsbehältnis
- Fig. 2: Seitenansicht der erfindungsgemäßen Darreichungsform im Kombinationsbehältnis mit zwei zweiten festen Komponenten
- Fig. 3: Seitenansicht der erfindungsgemäßen Darreichungsform im Kombinationsbehältnis mit zwei unterschiedlichen spezifischen Wirkstoffen enthalten in zwei zweiten festen Komponenten

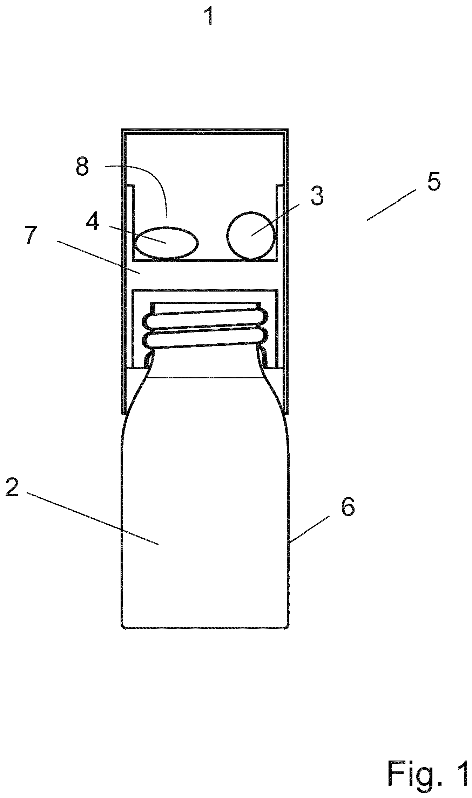

Die erfindungsgemäße Darreichungsform 1 (Fig. 1) umfasst eine flüssige Komponente 2, eine erste feste Komponente 3 sowie eine zweite feste Komponente 4 in Kapsel- und/oder Tablettenform eines Nahrungsergänzungsmittels für den menschlichen Verzehr. Die Komponenten sind in einem Kombinationsbehältnis verpackt, um die gemeinsame Anwendung der zusammengehörigen Komponenten zu ermöglichen. Das Kombinationsbehältnis umfasst ein erstes Behältnis 6, das dazu bestimmt ist, die flüssige Komponente 2 aufzunehmen. Dieses erste Behältnis 6 ist in Flaschenform ausgebildet und mittels eines Schraubverschlusses 7 verschließbar. Der Schraubverschluss 7 ist so ausgeführt, dass er ein zweites Behältnis 8 für die beiden festen Substanzen bildet, das durch einen Deckel 5 verschließbar ist. Das Nahrungsergänzungsmittel in diesem Ausführungsbeispiel hat die flüssige Komponente mit hochangereicherten Antioxidantien, eine erste feste Komponente mit einem Multivitaminkomplex und ein zweite feste Komponente mit einem spezifischen Wirkstoffkomplex zur Entgiftung des Körpers und der Stärkung des Immunsystems. Die flüssige Komponente 2 enthält sekundäre Pflanzenstoffe wie Polyphenole von Acerola und/oder Granatapfel, außerdem den Stabilisator Vitamin C in einer Konzentration von 9 g/l. Die genaue Zusammensetzung der flüssigen Komponente 2 ist in Tab. 1 dargelegt. Die erste feste Komponente 3 enthält einen Multivitaminkomplex auf organischer Basis, d.h, die Vitamine sind aus natürlich gewachsenen Pflanzen extrahiert. Der Multivitaminkomplex enthält alle essentiellen Vitamine, die der menschliche Körper benötigt und im Wesentlichen nicht selbst herstellen kann. Insbesondere enthält die erste feste Komponente 3 die Vitamine B5 und D sowie Mineralstoffe wie Calcium, Phosphor und/oder Kalium. Die genaue Zusammensetzung der ersten festen Komponente 3 ist in Tab. 1 dargelegt. Die zweite feste Komponente 4 ist optisch von der ersten festen Komponente 3 unterscheidbar und enthält produktspezifische Wirkstoffe, die je nach dem vom Anwender gewünschten Effekt kombiniert und dosiert sind. So können die Wirkstoffe der zweiten festen Komponente 4 Antioxidantien zu Entgiftung enthalten, Wirkstoffe, die das Immunsystem schützen oder Wirkstoffe zum Schutz der Haut oder Gefäßgesundheit enthalten. In diesem Ausführungsbeispiel enthält die zweite feste Komponente 4 Wirkstoffe, die Antioxidantien zur Entgiftung enthalten. Die genaue Zusammensetzung ist Tab. 1 zu entnehmen. Diese spezifischen Wirkstoffe unterstützen das Immunsystem und schützen den menschlichen Körper durch ihre antioxidative Wirkung vor Umweltschadstoffen.

**Tabelle 1: Zusammensetzung der flüssigen, der ersten und der zweiten festen Komponente für das Produkt "Immun & Detox": 1 Shot, 1 Kapsel Vitamine, 1 Kapsel Green Tea Extrakt**

| **Bestandteil** | **mg pro Kapsel** |
|---|---|
| **Flüssigkeit** | |
| Vitamin C | 200 |
| Acerola 25% | 800 |
| Antioxidantien | 1000 |
| Sirup | var. |
| Quellwasser | 22ml |

| **Vitaminkapsel** | |
|---|---|
| Vitamin B1 (Thiamin) | 1,82 |
| Vitamin B2 (Riboflavin | 1.89 |
| Vitamin B3 (Niacin) | 45.77 |
| Vitamin B5 | 9,43 |
| Vitamin B6 | 9,63 |
| Vitamin B9 (Folsäure) | 0.181 |
| Vitamin C | 393.7 |
| Vitamin E | 50.57 |
| Vitamin B7 (Biotin) | 046 |
| Iron | 7.61 |
| Magnesium | 1.318 |
| Zinc | 3.459 |
| Copper | 0,00094 |
| Chrome | 0,046 |
| Manganese | 1.03 |
| Potassium | 3.0 |
| Selenium | 0,029 |
| Calcium | 1.276 |
| Beta Carotene | 2.25 |
| Vitamin B3 (Niacin) | 15 |
| Vitamin D | 0.015 |

| **Wirkstoffkapsel** | |
|---|---|
| Polyphenole | 490 |
| Se | 5 |

Das Behältnis für die flüssige Komponente 2 besteht üblicherweise aus einer Aluminiumlegierung. Möglich ist auch ein fester Kunststoff, z.B. PP, oder Glas. Wichtig ist in diesem Zusammenhang, dass das Behältnis lichtundurchlässig gestaltet ist, um die Haltbarkeit der flüssigen Komponente 2 auch über einen längeren Zeitraum zu gewährleisten. Der Schraubverschluss 7 besteht aus einem festen Kunststoff, ebenso der Deckel 5, mit dem der Schraubverschluss 7 verschlossen ist, der gleichzeitig das Behältnis für die festen Komponenten bildet. Der Deckel 5, mit dem das Behältnis der festen Komponenten sicher verschlossen werden kann, ist so bemessen, dass er sich bei üblicher Handhabung durch den Anwender nicht lösen kann. Zugleich ist der Deckel 5 mit geringem Kraftaufwand abzunehmen.

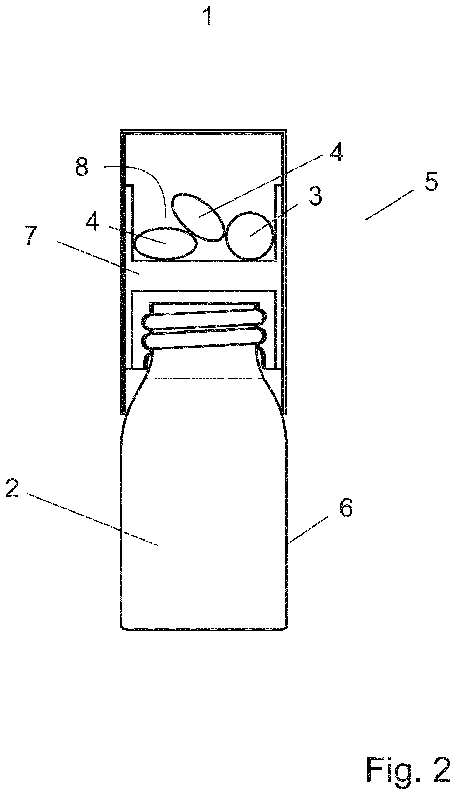

Fig. 2 zeigt eine Darreichungsform 1 in einem Kombinationsbehältnis, das zwei zweite feste Komponenten 4 enthält. Die Menge der spezifischen Wirkstoffe der zweiten festen Komponente 4 kann je nach Wahl der vom Anwender gewünschten Wirkung so hoch sein, dass die spezifischen Wirkstoffe auf zwei Kapseln bzw. Tabletten verteilt werden. Die Kapseln bzw. Tabletten sind so vom Anwender leichter einzunehmen und zu schlucken. Das Nahrungsergänzungsmittel in diesem Ausführungsbeispiel hat die flüssige Komponente mit hochangereicherten Antioxidantien, eine erste feste Komponente mit einem Multivitaminkomplex und ein zweite feste Komponente mit einem spezifischen Wirkstoffkomplex zur Reduzierung von Alterserscheinungen (Zusammensetzung s. Tab. 2). Die flüssige Komponente 2 mit sekundären Pflanzenstoffen befindet sich in dem flaschenförmigen ersten Behältnis 6. Das zweite Behältnis 8 verschraubt das erste Behältnis 6 und enthält die erste 3 und die zweite feste Komponente 4. Die erste feste Komponente 3 enthält einen Multivitaminkomplex und Mineralstoffe (Zusammensetzung s. Tab. 2). Die zweite feste Komponente 4 besteht aus zwei Kapseln bzw. Tabletten und enthält die spezifischen Wirkstoffe. Der Deckel 5 ist auf das zweite Behältnis 8 aufgesteckt.

**Tabelle 2: Zusammensetzung der flüssigen, der ersten und der zweiten festen Komponente für das Produkt "Beauty Antiageing": 1 Shot, 1 Kapsel Vitamine, 2 Kapseln Lycopin, Q10,Omega3**

| **Bestandteil** | **mg pro Kapsel** |
|---|---|
| **Flüssige Komponente** | |
| Vitamin C | 200 |
| Acerola 25% | 800 |
| Antioxidantien | 1000 |
| Sirup | var. |
| Quellwasser | 22 ml |

| **Vitaminkapsel** | |
|---|---|
| Vitamin B1 (Thiamin) | 0,788 |
| Vitamin B2 (Riboflavin | 0,823 |
| Vitamin B3 (Niacin) | 19,98 |
| Vitamin B5 | 4,01 |
| Vitamin B6 | 4,189 |
| Vitamin B9 (Folsäure) | 0,079 |
| Vitamin C | 171,18 |
| Vitamin E | 21,99 |
| Vitamin B7 (Biotin) | 0,02 |
| Iron | 3,312 |
| Magnesium | 0,573 |
| Zinc | 1,504 |
| Copper | 0,00041 |
| Chrome | 0,02 |
| Manganese | 0,448 |
| Potassium | 1,307 |
| Selenium | 0,013 |
| Calcium | 0,555 |
| Phosphor | 0,1 |
| Vitamin D | 0,015 |
| Beta Carotene | 2,25 |
| Vitamin B3 (Niacin) | |

| **Wirkstoffkapsel 1 + 2** | pro Kapsel |
|---|---|
| Lycopin | 15 |
| Se | 2,5 |
| Omega 3 | 138 |
| Coenzym 10 | 25 |

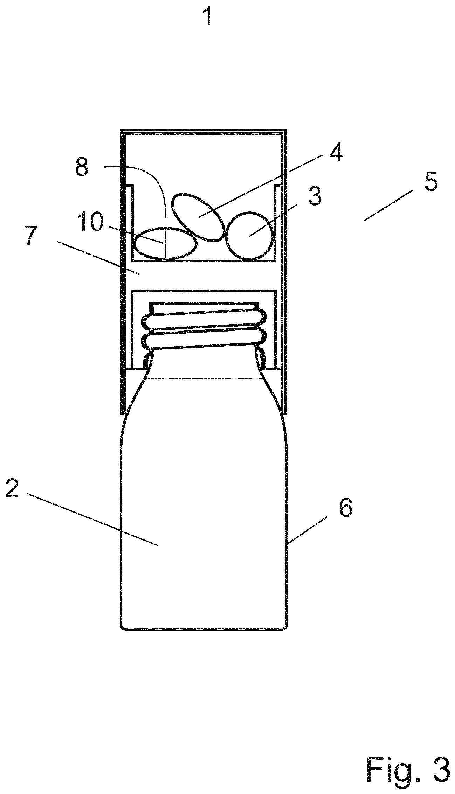

Im dritten Ausführungsbeispiel sind spezifische Wirkstoffe für das Produkt "Immun & Detox mit Beauty Antiageing" zur Reduzierung von Alterserscheinungen und Antioxidantien zu Entgiftung von Umweltschadstoffen kombiniert (Fig. 3). Die flüssige Komponente 2 mit sekundären Pflanzenstoffen befindet sich in dem flaschenförmigen ersten Behältnis 6. Das zweite Behältnis 8 verschraubt das erste Behältnis 6 und dient gleichzeitig als Behältnis für die erste 3 und die zweite feste Komponente 4. Der Deckel 5 ist auf das zweite Behältnis 8 aufgesteckt. Die erste feste Komponente 3 enthält den Multivitaminkomplex und Mineralstoffe (Zusammensetzung s. Tab. 3). Die zweite feste Komponente 4, 10 mit den spezifischen Wirkstoffen besteht aus zwei Kapseln bzw. Tabletten. Die erste Kapsel bzw. Tablette der zweiten festen Komponente 4 enthält spezifische Wirkstoffe zur Reduzierung von Alterserscheinungen (Zusammensetzung s. Tab. 3). Die zweite Kapsel bzw. Tablette der zweiten festen Komponente 10 enthält Antioxidantien zur Entgiftung von Umweltschadstoffen (Zusammensetzung s. Tab. 3). Alle drei Kapseln bzw. Tabletten 3, 4, 10 unterscheiden sich optisch voneinander, z.B. durch unterschiedliche Form- und/oder Farbgebung oder auch durch die Art (Tablette oder Kapsel). Auf diese Weise kann der Anwender zuverlässig erkennen, welche Komponente mit welchen Wirkstoffen er zu sich nimmt.

**Tabelle 3: Zusammensetzung der flüssigen, der ersten und der zweiten festen Komponente für das Produkt "Immun & Detox mit Beauty Antiageing": 1 Shot, 1 Kapsel Vitamine, 1 Kapsel Lycopin, Q10, Omega3 und 1 Kapsel Green Tea Extrakt**

| **Bestandteil** | **mg pro Kapsel** |
|---|---|
| **Flüssige Komponente** | |
| Vitamin C | 200 |
| Acerola 25% | 800 |
| Antioxidantien | 1000 |
| Sirup | Var. |
| Quellwasser | 22ml |

| **Vitaminkapsel** | |
|---|---|
| Vitamin B1 (Thiamin) | 0,788 |
| Vitamin B2 (Riboflavin | 0,823 |
| Vitamin B3 (Niacin) | 19,98 |
| Vitamin B5 | 4,01 |
| Vitamin B6 | 4,189 |
| Vitamin B9 (Folsäure) | 0,079 |
| Vitamin C | 171,18 |
| Vitamin E | 21,99 |
| Vitamin B7 (Biotin) | 0,02 |
| Iron | 3,312 |
| Magnesium | 0,573 |
| Zinc | 1,504 |
| Copper | 0,00041 |
| Chrome | 0,02 |
| Manganese | 0,448 |
| Potassium | 1,307 |
| Selenium | 0,013 |
| Calcium | 0,555 |
| Phosphor | 0,1 |
| Vitamin D | 0,015 |
| Beta Carotene | 2,25 |
| **Wirkstoffkapsel 1** | pro Kapsel |
| Lycopin | 15 |
| Se | 2,5 |
| Omega 3 | 138 |
| Coenzym 10 | 25 |

| **Wirkstoffkapsel 2** | |
|---|---|
| Polyphenole | 490 |
| Se | 5 |

### BEZUGSZEICHENLISTE

- 1: Nahrungsergänzungsmittels
- 2: flüssige Komponente
- 3: erste feste Komponente
- 4, 10: zweite feste Komponente
- 5: Kombinationsbehältnis
- 6: Erstes Behältnis
- 7: Schraubverschluss
- 8: Zweites Behältnis
- 9: Deckel

## Patentansprüche

1. Darreichungsform eines festen und flüssigen Nahrungsergänzungsmittels (1) wobei das Nahrungsergänzungsmittel einen Multivitaminkomplex, sekundäre Pflanzenstoffe und produktspezifische Wirkstoffe aufweist, umfassen
• eine flüssige Komponente (2)
• eine erste feste Komponente (3)
• eine zweite feste Komponente (4)

2. Darreichungsform eines festen und flüssigen Nahrungsergänzungsmittels (1) nach Anspruch 1
**dadurch gekennzeichnet, dass**
die erste feste Komponenten (3) in Form einer ersten Kapsel oder Tablette und die zweite feste Komponente (4) in Form einer zweiten Kapsel oder Tablette zusammengefasst.

3. Darreichungsform eines festen und flüssigen Nahrungsergänzungsmittels (1) nach Anspruch 1 oder 2
**dadurch gekennzeichnet, dass**
die flüssige Komponente (2) sekundäre Pflanzenstoffe und/oder Mineralstoffe aufweist.

4. Darreichungsform eines festen und flüssigen Nahrungsergänzungsmittels (1) nach Anspruch 3
**dadurch gekennzeichnet, dass**
die sekundären Pflanzenstoffe Acerolaextrakte und/oder Granatapfelextrakt und/oder andere sekundären Pflanzenstoffe aufweisen.

5. Darreichungsform eines festen und flüssigen Nahrungsergänzungsmittels (1) nach Anspruch 3 oder 4
**dadurch gekennzeichnet, dass**
die flüssigen Komponente (2) mindestens 100 mg, bevorzugt 250 mg und besonders bevorzugt 500 mg sekundäre Pflanzenstoffe aufweist.

6. Darreichungsform eines festen und flüssigen Nahrungsergänzungsmittels (1) nach einem oder mehreren der Ansprüche 3 bis 5
**dadurch gekennzeichnet, dass**
die flüssige Komponente (2) einen Stabilisator aufweist,
wobei der Stabilisator der flüssigen Komponente (2) organisches Vitamin C ist.

7. Darreichungsform eines festen und flüssigen Nahrungsergänzungsmittels (1) nach Anspruch 6
**dadurch gekennzeichnet, dass**
die flüssige Komponente (2) einen Anteil an organischem Vitamin C in einer Höhe zwischen 1 g/l und 15 g/l aufweist.

8. Darreichungsform eines festen und flüssigen Nahrungsergänzungsmittels (1) nach einem oder mehreren der Ansprüche 1 bis 7
**dadurch gekennzeichnet, dass**
die erste feste Komponente (3) einen Multivitaminkomplex aufweist.

9. Darreichungsform eines festen und flüssigen Nahrungsergänzungsmittels (1) nach Anspruch 8
**dadurch gekennzeichnet, dass**
der Multivitaminkomplex rein organischer Herkunft ist.

10. Darreichungsform eines festen und flüssigen Nahrungsergänzungsmittels (1) nach Anspruch 8 oder 9
**dadurch gekennzeichnet, dass**
der Multivitaminkomplex der ersten festen Komponente (3) Mineralstoffe aufweist.

11. Darreichungsform eines festen und flüssigen Nahrungsergänzungsmittels (1) nach Anspruch 10
**dadurch gekennzeichnet, dass**
die erste feste Komponente (3) mindestens 100 mg, bevorzugt 200 mg und besonders bevorzugt 300 mg Mineralstoffe und/oder Multivitaminkomplex aufweist.

12. Darreichungsform eines festen und flüssigen Nahrungsergänzungsmittels (1) nach einem oder mehreren der Ansprüche 1 bis 11
**dadurch gekennzeichnet, dass**
die zweite feste Komponente (4) produktspezifische Wirkstoffe aufweist.

13. Darreichungsform eines festen und flüssigen Nahrungsergänzungsmittels (1) nach Anspruch 12
**dadurch gekennzeichnet, dass**
die produktspezifischen Wirkstoffe der zweiten festen Komponente (4) wirksam zur Entgiftung, Schutz des Immunsystems, Hautschutz und/oder Gefäßgesundheit sind und/oder andere gesundheitsfördernde Wirkungen haben.

14. Darreichungsform eines festen und flüssigen Nahrungsergänzungsmittels (1) nach Anspruch 12 oder 13
**dadurch gekennzeichnet, dass**
die zweite feste Komponente (3) mindestens 50 mg, bevorzugt 150 mg und besonders bevorzugt mindestens 200 mg produktspezifischen Wirkstoff aufweist.

15. Darreichungsform eines festen und flüssigen Nahrungsergänzungsmittels (1) nach einem oder mehreren der Ansprüche 1 bis 14
**dadurch gekennzeichnet, dass**
die erste feste Komponente (3) und die zweite feste Komponente (4) optisch unterscheidbar sind.

16. Darreichungsform eines festen und flüssigen Nahrungsergänzungsmittels (1) nach einem oder mehreren der Ansprüche 1 bis 15
**dadurch gekennzeichnet, dass**
die Darreichungsform (1) in einem Kombinationsbehältnis (5) verpackt ist, dass ein erstes Behältnis (6) zur Aufnahme der flüssigen Komponente, einen Schraubverschluss (7), in den ein zweites Behältnis (8) Aufnahme der zwei festen Komponenten und einen Steckdeckel (9) aufweist,
wobei das erste Behältnis (6) durch den Schraubverschluss (7) verschließbar ist und wobei das zweite Behältnis (8) durch den Steckdeckel (9) verschließbar ist.

17. Darreichungsform eines festen und flüssigen Nahrungsergänzungsmittels (1) nach einem oder mehreren der Ansprüche 1 bis 16
**dadurch gekennzeichnet, dass**
die Mineralstoffe, die sekundären Pflanzenstoffe und/oder die produktspezifischen Wirkstoffe rein organischer Herkunft sind.
